# EUROPEAN PATENT APPLICATION

(11) **EP 0 882 461 A2**
(43) Date of publication of application: **09.12.1998**
(21) Application number: 98110173.6
(22) Date of filing: 04.06.1998
(51) Int. Cl.: A61L 29/00, A61L 31/00, A61L 27/00

(54) **Medical device and production method thereof**

(30) Priority: 04.06.1997 JP 146220/97
(71) Applicant: UNITIKA LTD., Amagasaki-shi, Hyogo 660-0824 (JP)
(72) Inventor: Kimura, Takashi, c/o Unitika Ltd., Uji-shi, Kyoto (JP); Takagi, Kunihiko, c/o Unitika Ltd., Uji-shi, Kyoto (JP)
(74) Representative: Hansen, Bernd, Dr. Dipl.-Chem.

(57) **Abstract**

A medical device having both physiological and antimicrobial activity, comprising a base material, a cross-linked coating film formed on a surface of the base material, and each of a physiologically active substance and an antimicrobial substance bonded to the coating film. Also disclosed is a method for producing the medical device, which comprises providing a base material, forming a cross-linked coating film comprising the reaction product of a high molecular weight substance having acid anhydride groups and a compound having two or more active hydrogen atoms on a surface of the base material, and subsequently bonding each of a physiologically active substance and an antimicrobial substance to the coating film.

## Description

### FIELD OF THE INVENTION

This invention relates to a medical device which simultaneously exhibits both physiological and antimicrobial activity, and to a method for producing the medical device.

### BACKGROUND OF THE INVENTION

Proposals have been made to develop medical materials having various functions by attaching to the surface thereof enzymes, polysaccharides, coenzymes, enzyme inhibitors, hormones, antigens, antibodies and the like physiologically active substances. For example, JP-A-50-139174 (the term "JP-A" as used herein means an "unexamined published Japanese patent application") discloses a method in which a high molecular weight substance having hydroxyl groups is reacted with an aminoaldehyde, aminoacetal or the like to introduce amino groups into the high molecular weight substance which is then reacted with heparin. Also, JP-B-53-15913 (the term "JP-B" as used herein means an "examined Japanese patent publication") discloses a method for imparting fibrinolytic activity in which a fibrinolytic enzyme is linked to the surface of a polyamide. In addition, Japanese Patent No. 1406830 discloses a method in which enzyme activity is added to the surface of a solid material.

On the other hand, cases have been increasing in recent years in which a treatment or diagnosis is carried out by percutaneously inserting and indwelling a catheter or the like medical device into the living body. However, such procedures also cause a serious problem of inducing infectious diseases mediated by the catheter and the like medical devices. For example, a vessel catheter or a ureteral catheter is indwelt in the living body for a prolonged period of time in many cases, and bacteria invade through these catheters and frequently cause sepsis, urethritis, cystitis, pyelitis and the like symptoms. Because of this, washing of the inserting part of each medical device or injection of a germicide is carried out as a means for preventing infectious diseases caused by a catheter and the like medical devices. However, not only is such handling complex, but also the handling itself becomes a new source of infection.

Although the preventive administration of antibiotics and chemotherapeutic drugs is also carried out, it is said that unplanned chemotherapy is harmful because of the problems of inducing side effects and increasing the generation frequency of resistant strains. Also, these antibiotics and chemotherapeutic drugs are meant to be topically applied. With regard to the topical use of antibiotics and chemotherapeutic drugs, JP-B-2-25625 discloses a ureteral catheter having the ability to prevent urinary tract infection, in which antibiotics are linked through an ionic bonding to a ureteral catheter made of an olefinic polymer, a diene polymer or a silicon polymer as a raw material. JP-B-56-34203 discloses a method for producing an antimicrobial material in which a high molecular weight substance having acidic groups is allowed to react with bis-(p-chlorophenyldiguanido)-hexane or a salt thereof. Additionally, JP-W-7-501470 (the term "JP-W" as used herein means an "published Japanese national phase international patent application") discloses antimicrobial medical articles coated with antimicrobial substances.

As described above, there are reports on medical devices in which a physiological activity or an antimicrobial activity is separately provided, but virtually nothing is known about a medical material and a method for producing the same in which not only a physiologically active function is expressed by immobilizing a physiologically active substance but also an antimicrobial function is provided by ionic bonding of an antimicrobial substance.

### SUMMARY OF THE INVENTION

It is therefore an object of the present invention to solve the aforementioned problems of the prior art, and to thereby provide a medical device which simultaneously exhibits both excellent physiological and antimicrobial activity and a method for producing the same.

With the aim of resolving the aforementioned problems, the present inventors have conducted extensive studies and found as a result of their efforts that a medical device which has both a physiological activity and an antimicrobial activity at the same time can be obtained providing a base material which constitutes the medical device, allowing a high molecular weight substance having acid anhydride groups to react with a compound having two or more active hydrogen atoms on the surface of the base material to form a cross-linked coating film on the surface of the base material, subsequently linking a physiologically active substance via covalent bonds to acid anhydride groups which are present on the cross-linked coating film, and also linking an antimicrobial substance via ionic bonds to carboxyl groups formed by hydrolysis of the acid anhydride groups. The present invention has been accomplished on the basis of these findings.

Accordingly, a first aspect of the present invention is directed to a medical device having both a physiological and antimicrobial activity, comprising a base material, a cross-linked coating film formed on a surface of the base material, and each of a physiologically active substance and an antimicrobial substance bonded to the coating film. A second aspect of the present invention is directed to a method for producing a medical device having both a physiological and antimicrobial activity, which comprises providing a base material forming a cross-linked coating film comprising the reaction product of a high molecular weight substance having acid anhydride groups and a compound having two or more active hydrogen atoms on a surface of the base material, and subsequently bonding each of a physiologically active substance and an antimicrobial substance to the coating film.

According to the present invention, a medical device having functions of both physiological activity and antimicrobial activity can be obtained by a simple and easy method.

Other objects and advantages of the present invention will become apparent in view of the followed detailed description and working examples.

### DETAILED DESCRIPTION OF THE INVENTION

The medical device according to the present invention, although not particularly limited, is a device which is mainly used for indwelling in the living body, and illustrative examples thereof include an IVH catheter, a thermodilution catheter, an angiography catheter, a vasodilation catheter, an artificial blood vessel, a shunt tube, a canula, a dilator, an indwelling needle, a guide wire, various sensors for use in the measurement of blood flow and blood gas concentration and the like medical devices which are inserted into or indwelt in blood vessels; a ureteral catheter and the like medical devices which are inserted into or indwelt in the urinary tract or ureter; a tracheotomy tube, an endotracheal tube and the like medical devices which are inserted into or indwelt in the trachea; and a nasal feeding tube, a nutrition catheter, a feeding gastric tube and the like medical devices which are inserted or indwelt orally or transnasally; as well as an artificial kidney, an artificial heart, an artificial valve, an artificial lung and the like medical devices.

Although not particularly limited, the base material which constitutes the medical device preferably comprises a high molecular weight material such as polyurethane, polyamide, polyester, polyvinyl chloride, polyethylene, polypropylene, polystyrene, polyacrylic acid ester, polymethacrylic acid ester, polycarbonate, polyacrylonitrile, polyacrylamide, polyacrylic acid, polymethacrylic acid, polyvinyl alcohol, polymaleic anhydride, polyethyleneimine, cellulose, natural rubber or the like organic substance or silicone resin, glass, asbestos, mica, activated carbon, polyphosphazene or the like inorganic substance. In the case of a medical device composed of a metal or the like material other than a high molecular weight material, a cross-linked coating film can be formed directly on its surface, but it is effective and desirable to first coat the base material with the aforementioned high molecular weight material and to then form a cross-linked coating film on the surface of the high molecular weight material.

According to the medical device of the present invention, a cross-linked coating film is formed on the surface of the base material, and a physiologically active substance and an antimicrobial substance are bonded to the thus formed cross-linked coating film. This cross-linked coating film can be obtained by allowing a high molecular weight substance having acid anhydride groups to react with a compound having two or more active hydrogen atoms in accordance with a method which will be described below.

The high molecular weight substance having acid anhydride groups for use in the present invention is a polymer or copolymer which contains at least two monomer units, each having an acid anhydride group, in one molecule, and illustrative examples thereof include maleic anhydride-ethylene copolymer, maleic anhydride-styrene copolymer, maleic anhydride-methyl vinyl ether copolymer and the like maleic anhydride polymers, polyacrylic anhydride, acrylic anhydride-styrene copolymer and the like acrylic anhydride polymers, and polymethacrylic anhydride, methacrylic anhydride-styrene copolymer and the like methacrylic anhydride polymers. The molecular weight of the high molecular weight substance having acid anhydride groups is not particularly limited, but is generally 500 or more, preferably 750 or more, more preferably 1,000 or more.

The compound having two or more active hydrogen atoms for use in the present invention is a compound having at least two groups selected from a hydroxyl group, an amino group, a thiol group and the like active hydrogen groups in one molecule, and illustrative examples thereof include a polyol, a polyamine and the like compounds.

The polyol is a low molecular weight or high molecular weight compound having at least two hydroxyl groups in one molecule, and illustrative examples thereof include ethylene glycol, propylene glycol, butylene glycol, glycerol, pentaerythritol, sorbitol, diglycerol, diethylene glycol, triethylene glycol, tetraethylene glycol, pentaethylene glycol, dipropylene glycol, tripropylene glycol, polyethylene glycol, polypropylene glycol, polybutylene glycol and the like, of which a polyalkylene glycol is particularly preferred.

The polyamine is a low molecular weight or high molecular weight compound having at least two amino groups in one molecule, and illustrative examples thereof include ethylenediamine, propylenediamine, hexamethylenediamine, poly (oxyethylene)diamine, poly(oxypropylene)diamine and the like diamines, polyvinylamine, aminoacetalized polyvinyl alcohol, polyethyleneimine and a reaction product of a diamine with epichlorohydrin.

According to the medical device of the present invention, a physiologically active substance is linked via covalent bonds to unreacted acid anhydride groups which are present in the cross-linked coating film, and an antimicrobial substance is linked via ionic bonds to carboxyl groups formed by the hydrolysis of the acid anhydride groups.

The physiologically active substance according to the present invention is a substance which has a physiological activity other than antimicrobial activity, and it must have amino group in its structure. Illustrative examples of such a substance include drugs, coenzymes, antibodies, receptors, antigens, complements, hormones, enzymes such as deoxyribonuclease, a blood proteolytic enzyme and the like, enzyme substrates, enzyme inhibitors and blood proteins such as albumin, globulin, ceruloplasmin, transferrin, cycloglobulin, haptoglobulin, hemopexin, conglutinin and the like, of which streptokinase, urokinase, plasmin, brinolase, tissue plasminogen activator and the like fibrinolytic enzymes are particularly preferred.

The antimicrobial substance for use in the present invention is preferably a basic antimicrobial substance, and illustrative examples thereof include amoxicillin, ampicillin, ciclacillin, sultamicillin tosilate, talampicillin hydrochloride, bacampicillin hydrochloride, hetacillin potassium, lenampicillin hydrochloride, pivmecillinam hydrochloride, aspoxicillin and the like penicillin antibacterial drugs; cefaclor, cefatrizine propylene glycol, cefadroxil, cefalexin, cefaloglycin, cefradine, cefroxadine, cefazolin sodium, cefapirin sodium, cefaloridine, ceftezole sodium, cefotiam hydrochloride, cefamandole sodium, cefametazole sodium, cefasulodin sodium, cefmenoxime hydrochloride, cefotaxime sodium, cefotetan, cefoperazone sodium, ceftazidime, ceftizoxime sodium, ceftriaxone sodium, cefpimizole sodium, cefpiramide sodium, cefbuperazone sodium, latamoxef sodium, cefixime, cefteram pivoxyl, cefminox sodium, cefuzonam sodium and the like cephalosporin antibacterial drugs; gentamicin sulfate, netilmicin sulfate, tobramycin, amikacin sulfate, streptomycin sulfate, fradiomycin sulfate, bekanamycin sulfate, paromomycin sulfate, ribostamycin sulfate, kanamycin sulfate, dibekacin sulfate, sisomicin sulfate, micronomicin sulfate, astromicin sulfate and the like aminoglycoside antibacterial drugs; colistin sulfate, polymyxin B sulfate and the like polypeptide antibacterial drugs; oxytetracycline, tetracycline, demethylchlor tetracycline, doxycycline hydrochloride, minocycline hydrochloride, rolitetracycline and the like tetracycline antibacterial drugs; lincomycin hydrochloride, clindamycin hydrochloride, clindamycin palmitate hydrochloride, clindamycin phosphate and the like lincomycin antibacterial drugs; erythromycin, erythromycin estolate, erythromycin ethylsuccinate, oleandomycin phosphate, triacetyloleandomycin, kitasamycin, acetylkitasamycin, acetylspiramycin, josamycin, josamycin propionate, midecamycin, midecamycin acetate, rokitamycin and the like macrolide antibacterial drugs; thiamphenicol aminoacetate hydrochloride and the like chloramphenicol antibacterial drugs; cycloserine, rifampicin, capreomycin sulfate, enviomycin sulfate and the like antituberculous drugs; nystatin, amphotericin B and the like antimycotic drugs; vancomycin hydrochloride and the like glycopeptide antibacterial drugs; and aztreonam, spectinomycin hydrochloride, imipenem-cilastatin sodium and the like antibiotics.

Also useful as the antimicrobial substance of the present invention includes sulfisomidine, sulfamethizole, sulfamethoxazole, acetylsulfamethoxazole, sulfamonomethoxine, sulfadimethoxine, sulfaphenazole, sulfamethopyrazine, salazosulfapyridine and the like sulfa drugs; calcium p-aminosalicylate, calcium alumino-p-aminosalicylate, isoniazid, isoniazid sodium methanesulfonate, isoniazid sodium glucuronate, isoniazid calcium pyruvate, pyrazinamide, ethionamide, protionamide, ethambutol hydrochloride and the like antituberculous agents; glucosulfone sodium, diaphenylsulfone, thiazosulfone, and the like antileprotics; nitrofurantoin and the like furan preparations; nalidixic acid, piromidic acid, pipemidic acid trihydrate, cinoxacin, enoxacin, ofloxacin, norfloxacin and the like pyridone carboxylic acid preparations; flucytosine, miconazole and the like antimycotic agents; aciclovir, vidarabine and the like antiviral agents; and trimethoprim and the like chemotherapeutic agents having basic functional groups. In addition, chlorhexidine gluconate, benzalkonium chloride and the like dermatologic germicidal disinfectants having basic functional groups can also be used as the antimicrobial substance of the present invention. Although these antimicrobial substances may exist in various salt forms in addition to their free forms, any one of these forms can be used as the antimicrobial substance of the present invention as a matter of course. These antimicrobial substances may be used alone or as a mixture thereof.

Based on the construction described above, the medical device of the present invention simultaneously expresses both physiological and antimicrobial activities.

Next, the method for producing a medical device as a second aspect of the present invention is described below.

Firstly, a cross-linked coating film is formed on the surface of the medical device base material as described above, by allowing the aforementioned high molecular weight substance having acid anhydride groups to react with the aforementioned compound having two or more active hydrogen atoms. In order to effect this reaction, the surface of the base material of a medical device is contacted with a solution in which the high molecular weight substance having acid anhydride groups and the compound having two or more active hydrogen atoms are dissolved.

Examples of the solvent for use in dissolving the high molecular weight substance having acid anhydride groups and the compound having two or more active hydrogen atoms include dioxane, tetrahydrofuran, ethyl acetate, acetone, methyl ethyl ketone, chloroform, nitromethane, benzene, toluene, xylene, dimethylformamide, dimethylacetamide, dimethyl sulfoxide and the like solvents.

The high molecular weight substance having acid anhydride groups is dissolved in the above described solvent in an amount of 0.1% by weight or more, preferably from 0.2 to 10% by weight, more preferably from 0.5 to 5.0% by weight. A polyol, polyamine or the like compound having two or more active hydrogen atoms is further dissolved therein in an amount of 0.001% by weight or more, preferably from 0.005 to 10.0% by weight, more preferably from 0.01 to 5.0% by weight. If the amount of each of these materials dissolved in the solvent is too low, then this would reduce the strength of the coating film thus formed, and if too high, would cause unevenness in the thickness of the coating film.

The ratio of the high molecular weight substance having acid anhydride groups to the compound having two or more active hydrogen atoms in the above described solution may be from 1:1 to 10,000:1, preferably from 10:1 to 5,000:1, more preferably from 100:1 to 1,000:1, as a molar ratio of the acid anhydride groups to the functional groups containing active hydrogen atoms. A case in which the amount of the acid hydride groups is smaller than that of the functional groups containing active hydrogen atoms is not desirable. This is because the amount of unreacted acid anhydride groups remaining after the cross-linking reaction is small such that it becomes difficult to effect bonding of the physiologically active substance. Also, a case in which the amount of the functional groups containing active hydrogen atoms is too small in comparison with that of the acid hydride groups is also not desirable. This is because the strength of the cross-linked coating film becomes poor which can cause the coating film to separate or break apart.

The method for contacting the thus prepared solution with the surface of a base material is not particularly limited. Examples thereof include a method in which a medical device is soaked in the aforementioned solution, a method in which the solution is sprayed on the surface of the base material and a method in which the solution is coated on the surface of the base material. Of these, the method in which a medical device is soaked in the solution is particularly preferred because of its good reproducibility.

In the soaking method, the soaking time may be preferably from 10 seconds to 24 hours, more preferably from 30 seconds to 2 hours.

After contacting the solution with the surface of a base material, a cross-linked coating film is formed by allowing the base material to stand at a temperature of preferably from -50 to 180°C, more preferably from 0 to 150°C, for a period of from 5 minutes 72 hours, preferably from 10 minutes to 48 hours, more preferably from 30 minutes to 24 hours.

Next, a physiologically active substance and an antimicrobial substance are linked to the medical device on which a cross-linked coating film has been formed. Prior to carrying out this step, however, it is desirable to soak the medical device in a solvent capable of dissolving both the high molecular weight substance having acid anhydride groups and the compound having two or more active hydrogen atoms, in order to further improve the physiological activity that is to be added to the medical device.

The aforementioned solvents such as dioxane, tetrahydrofuran, ethyl acetate, acetone, methyl ethyl ketone, chloroform, nitromethane, benzene, toluene, xylene, dimethylformamide, dimethylacetamide, dimethyl sulfoxide and the like can be used as the solvent for this purpose.

The soaking time for improving the physiological activity is preferably from 10 seconds to 24 hours, and the soaking temperature is preferably from -50 to 180°C.

Although the mechanism of improving physiological activity by such treatments is not clear, it is considered that a solvent capable of dissolving both of the two different molecules which form the cross-linked coating film easily permeates into the cross-linked coating film. This causes swelling of the cross-linked coating film to increase the degree of freedom of the molecular chains, so that water molecules can penetrate easily into the cross-linked coating film when used in an aqueous medium. Also, the physiologically active substance on the base material surface can easily adopt a conformation suitable for the expression of its activity.

With regard to the method for linking a physiologically active substance to the cross-linked coating film, the solution containing the aforementioned physiologically active substance may be sprayed or coated on the medical device of interest having a cross-linked coating film formed thereon. In the case of a tubular medical device such as a catheter, a solution containing a physiologically active substance having amino groups may be circulated in the tube, but a method in which a medical device is soaked in a solution containing a physiologically active substance is most simple and easy.

The solvent for use in this case is not particularly limited, with the proviso that it can dissolve the physiologically active substance and is free from amino groups. However, it is desirable to use an aqueous solution which is adjusted to a pH value of from 3 to 10 when a fibrinolytic enzyme is linked to the coating film. The amount of the physiologically active substance to be dissolved in this solution varies depending, for example, on the type of each physiologically active substance and the shape of each medical device. In the case of a fibrinolytic substance, the solution may be prepared such that its concentration per unit area of a medical device is 10 to 1,000 units/cm². Preferably, the soaking may be carried out for a period of from 1 to 48 hours at a temperature of from 0 to 80°C.

With regard to the ionic bonding of an antimicrobial substance, the solution containing the aforementioned antimicrobial substance may be sprayed or coated or, in the case of a tubular medical device such as a catheter, a solution containing an antimicrobial substance may be circulated in the tube. However, a method in which a medical device is soaked in a solution of an antimicrobial substance is the most simple and easy method.

The solvent for use in this case is not particularly limited, with the proviso that it can dissolve the antimicrobial substance, but water is most preferred. Preferably, the antimicrobial substance is used in a concentration of from 0.01 to 10% by weight, and the soaking may be carried out for a period of from 5 minutes to 48 hours at a temperature of from 0 to 80°C.

In carrying out ionic bonding of an antimicrobial substance, it is efficient and therefore desirable to adjust the pH value of the antimicrobial substance solution within a specified range. That is, the solution is maintained under a weakly acidic to alkaline condition, illustratively at a pH value of from 4 to 14. In order to adjust the pH value, an acid or alkali may be added dropwise, or a buffer solution having an appropriate concentration may be used. The antimicrobial substance thus linked via ionic bonding to the cross-linked coating film of a medical device is maintained on the coating film for a prolonged period of time and keeps its antimicrobial activity during use.

As described above, a physiologically active substance and an antimicrobial substance may be linked in order, but it is possible as a matter of course to simultaneously link the physiologically active substance and the antimicrobial substance to the cross-linked coating film. With regard to a method for simultaneous linking, a solution containing the physiologically active substance and the antimicrobial substance may be sprayed or coated on the medical device of interest having formed thereon a cross-linked coating film. In the case of a tubular medical device such as a catheter, the solution may be circulated in the tube, but a method in which a medical device is soaked in the solution is the most simple and easy method. Preferably, the soaking may be carried out for a period of from 1 to 48 hours at a temperature of from 0 to 80°C.

Because these methods for carrying out ionic bonding of an antimicrobial substance to the medical device are markedly easy and simple, they can be practiced easily by doctors, pharmacists, nurses and the like persons engaged in medical treatments at hospitals, drugstores and the like places where health control is practiced. In that case, doctors, pharmacists, nurses and the like persons engaged in medical treatments can prepare a made-to-order medical device in a hospital by selecting an appropriate antimicrobial substance in response to the actual circumstances of the hospital, such as the kinds of infectious bacteria and the like. Furthermore, depending on each type of disease, such persons may carry out ionic bonding of the thus selected antimicrobial substance to a physiologically active substance-linked medical device.

### EXAMPLES

Examples of the present invention are given below by way of illustration and not by way of limitation.

### Reference Example 1

### [Test of antimicrobial activity]

The antimicrobial activity of each sample was tested by the following diffusion method.

Firstly, a catheter tube to be tested was cut to a length of 5 mm and used as a sample. Thereafter, the sample was placed on an agar plate medium on which cells of *Staphylococcus aureus* ATCC 29523 had been spread. The resulting plate was incubated at 37°C for 24 hours, and then the size of the growth inhibition zone formed around the sample was measured. This was used as an index of antimicrobial activity ("Bioassay of Physiologically Active Substances", issued by Kodan-sha, p. 18).

### Reference Example 2

### [Test on fibrinolytic activity]

The fibrinolytic activity of each sample was tested by the method of Kanai *et al*. ("A Manual of Clinical Inspection Methods", revised 27th edition, issued by Kanehara Shuppan, VI-100). Namely, a sample was placed on a fibrin plate and incubated at 37°C for 24 hours, and then lysis of the fibrin membrane around the disc was observed.

### Reference Example 3

### [Test on urokinase activity]

The urokinase activity of each sample was tested by a synthetic substrate method (Morita *et al*., *J. Biochem*., 82, 1495 (1977)).

Namely, a catheter tube having a length of 1 cm was soaked in Tris-HCl buffer (pH 8.0) containing Glt-Gly-Arg-MCA (mfd. by Peptide Research Institute) and a reaction for a period of 10 minutes was carried out at 37°C. After completing the reaction, the activity of urokinase covalently bonded to the sample were obtained by measuring the fluorescence intensity (Ex., 380 nm; Em., 460 nm) of the reaction solution.

### Example 1

Firstly, 5% by weight of a maleic anhydride-methyl vinyl ether copolymer having a molecular weight of about 67,000 (mfd. by I. S. P.) and 0.1% by weight of polyethylene glycol having an average molecular weight of 3,000 (mfd. by Maruzen Pharmaceutical) were dissolved in acetone. Next, a catheter tube made of nylon 6 (5.6 mm in inner diameter and 8.0 mm in outer diameter, mfd. by UNITIKA) was soaked in the acetone solution for 1 hour at room temperature and then removed from the acetone solution and air-dried. The catheter tube was then heated at 90°C for 3 hours under reduced pressure to form a cross-linked coating film thereon. The catheter tube on which a cross-linked coating film had been formed was soaked in an aqueous solution containing 0.1 mg/ml of streptokinase (mfd. by Nakalai Tesque) and 1 mg/ml of kanamycin sulfate (mfd. by Wako Pure Chemical Industries) for 24 hours and then dried, to thereby obtain a medical device of the present invention having both fibrinolytic and antimicrobial activities.

The antimicrobial activity and fibrinolytic activity of this medical device were evaluated by the methods of Reference Examples 1 and 2. It was confirmed that the device possessed both of these functions.

### Example 2

Firstly, a catheter tube having an inner diameter of 1.8 mm and an outer diameter of 2.2 mm was obtained by subjecting polyurethane (Pellethane, mfd. by Dow Chemical) to extrusion molding at 180°C.

Next, 5% by weight of a maleic anhydride-methyl vinyl ether copolymer having a molecular weight of about 67,000 (mfd. by I. S. P.) and 0.1% by weight of polyethylene glycol having an average molecular weight of 3,000 (mfd. by Maruzen Pharmaceutical) were dissolved in acetone. The above prepared polyurethane catheter tube was soaked in the acetone solution for 1 minute at room temperature and then heated at 70°C for 24 hours to form a cross-linked coating film. The catheter tube having thereon the cross-linked coating film was then soaked in physiological saline containing 600 units/ml of urokinase (mfd. by The Green Cross Corporation) for 24 hours at 7°C and then dried. Thereafter, the thus treated catheter tube was again soaked in an aqueous solution containing 1 mg/ml of vancomycin hydrochloride (mfd. by Nakalai Tesque) for 1 hour at room temperature and then dried, to thereby obtain a medical device of the present invention having both urokinase activity and antimicrobial activity.

The antimicrobial activity and urokinase activity of the thus obtained catheter tube were evaluated by the methods of Reference Examples 1 and 3. It was confirmed that the tube possessed both of these activities.

### Comparative Example 1

In Example 2, a catheter tube to which urokinase was linked but vancomycin hydrochloride was not linked was prepared for the sake of comparison, and was found to have a urokinase activity similar to that of Example 2. This result suggests that the urokinase activity is not affected by the presence or absence of bound vancomycin hydrochloride.

### Comparative Examples 2 and 3

In Example 2, a catheter tube to which urokinase was not linked but vancomycin hydrochloride was linked was prepared for comparison (Comparative Example 2). Also, another catheter tube was prepared by repeating the procedure of Example 2 except that polyethylene glycol was not used (Comparative Example 3). Each of the catheter tubes of Example 2 and Comparative Examples 2 and 3 was soaked in a 1 M sodium chloride aqueous solution, and the amount of vancomycin released in the supernatant was measured by liquid chromatography (column: Cosmosil 5C18-MS mfd. by Nakalai Tesque, 10% acetonitrile, detection: 254 nm). As a result, the adsorbed amount of vancomycin was 121 µg/cm² in the tube of Example 2 and 120 µg/cm² in the tube of Comparative Example 2, but no vancomycin adsorption was observed in the tube of Comparative Example 3. Based on these results, it was found that the amount of adsorbed vancomycin is hardly affected by the presence or absence of bound urokinase, and that adsorption of vancomycin is due to the addition of a cross-linking agent.

### Examples 3 to 6

Firstly, a catheter tube having an inner diameter of 5.6 mm and an outer diameter of 8.0 mm was obtained by subjecting polyethylene terephthalate (mfd. by UNITIKA) to extrusion molding at 180°C.

Next, 5% by weight of a maleic anhydride-methyl vinyl ether copolymer having a molecular weight of about 67,000 (mfd. by I. S. P.) and 0.1% by weight of poly(oxyethylene)diamine having an average molecular weight of 3,000 (mfd. by Sanyo Kasei Kogyo) were dissolved in acetone. The above obtained catheter tube was soaked in the acetone solution for 1 minute at room temperature and then air-dried and allowed to stand at room temperature to form a cross-linked coating film thereon. The catheter tube having a coating film thereon was soaked in physiological saline containing 600 international units/ml of urokinase (mfd. by The Green Cross Corporation) for 24 hours at 7°C and then dried. Thereafter, the thus treated catheter tube was again soaked in an aqueous solution containing 1 mg/ml of cefazolin sodium (Example 3), amikacin sulfate (Example 4), minocycline hydrochloride (Example 5) or rifampicin (Example 6) for 1 hour at room temperature and then dried, to thereby obtain four different medical devices of the present invention having both urokinase activity and antimicrobial activity.

The antimicrobial and urokinase activities of the thus obtained catheter tubes were evaluated by the methods of Reference Examples 1 and 3. It was confirmed that these four tubes possessed the respective antimicrobial and urokinase activities.

While the invention has been described in detail and with reference to specific examples thereof, it will be apparent to one skilled in the art that various changes and modifications can be made therein without departing from the spirit and scope thereof.

## Claims

1. A medical device having both physiological and antimicrobial activity, comprising a base material, a cross-linked coating film formed on a surface of the base material, and each of a physiologically active substance and an antimicrobial substance bonded to the coating film.

2. The medical device as claimed in claim 1, wherein said physiologically active substance is covalently bonded to the cross-linked coating film and said antimicrobial substance is ionically bonded to the cross-linked coating film.

3. The medical device as claimed in claim 1, wherein said cross-linked coating film comprises the reaction product of a high molecular weight substance having acid anhydride groups and a compound having two or more active hydrogen atoms.

4. The medical device as claimed in claim 3, wherein said physiologically active substance is covalently bonded to unreacted acid anhydride groups present in the cross-linked coating film and said antimicrobial substance is ionically bonded to carboxyl groups formed by hydrolysis of acid anhydride groups present in the cross-linked coating film.

5. The medical device as claimed in claim 2, wherein said antimicrobial substance is a basic antimicrobial substance.

6. The medical device as claimed in claim 3, wherein said antimicrobial substance is a basic antimicrobial substance.

7. The medical device as claimed in claim 3, wherein said high molecular weight substance having acid anhydride groups is selected from the group consisting of maleic anhydride polymers, acrylic anhydride polymers and methacrylic anhydride polymers and said compound having two or more active hydrogen atoms is selected from the group consisting of polyols and polyamines.

8. The medical device as claimed in claim 1, wherein said physiologically active substance is selected from the group consisting of drugs, coenzymes, antibodies, receptors, antigens, complements, hormones, enzymes, enzyme substrates, enzyme inhibitors and blood proteins.

9. The medical device as claimed in claim 1, wherein said physiologically active substance is selected from the group consisting of streptokinase, urokinase, plasmin, brinolase and tissue plasminogen activator.

10. The medical device as claimed in claim 1, wherein said antimicrobial substance is selected from the group consisting of penicillin antibacterial drugs, cephalosporin antibacterial drugs, aminoglycoside antibacterial drugs, polypeptide antibacterial drugs, tetracycline antibacterial drugs, lincomycin antibacterial drugs, macrolide antibacterial drugs, chloramphenicol antibacterial drugs, antituberculosis drugs, antimycotic drugs, glycopeptide antibacterial drugs, sulfa drugs, antileprotics, furan preparations, pyridone carboxylic acid preparations, antiviral agents having basic functional groups, chemotherapeutic agents having basic functional groups and dermatologic germicidal disinfectants having basic functional groups.

11. A method for producing a medical device having both physiological and antimicrobial activity, which comprises providing a base material, forming a cross-linked coating film comprising the reaction product of a high molecular weight substance having acid anhydride groups and a compound having two or more active hydrogen atoms on a surface of the base material, and subsequently bonding each of a physiologically active substance and an antimicrobial substance to the coating film.

12. The method as claimed in claim 11, wherein said step of forming a cross-linked coating film comprises preparing a solution of a high molecular weight substance having acid anhydride groups and a compound having two or more active hydrogen atoms, and then contacting the base material with said solution.

13. The method as claimed in claim 11, which comprises preparing a solution of the high molecular weight substance having acid anhydride groups and the compound having two or more active hydrogen atoms in an amount of 0.1% by weight or more and 0.001% by weight or more, respectively, wherein the molar ratio of acid anhydride groups to functional groups containing an active hydrogen atom in said solution is from 1:1 to 10,000:1, and then contacting the base material with said solution.

14. The method as claimed in claim 11, further comprising the step of contacting the coated base material with a solvent capable of dissolving both the high molecular weight substance having acid anhydride groups and the compound having two or more active hydrogen atoms prior to bonding a physiologically active substance and an antimicrobial substance to the coating film.

15. The method as claimed in claim 11, wherein said step of bonding a physiologically active substance to the coating film comprises contacting the coated base material with a solution of said physiologically active substance.

16. The method as claimed in claim 11, wherein said step of bonding an antimicrobial substance to the coating film comprises contacting the coated base material with an aqueous solution of said antimicrobial substance having a pH of from 4 to 14.

17. The method as claimed in claim 11, which comprises simultaneously bonding the physiologically active substance and the antimicrobial substance to the coating film.

18. The method as claimed in claim 11, which comprises contacting the coated base material with a single solution of said physiologically active substance and said antimicrobial substance.

19. The medical device as claimed in claim 1 for indwelling in a living body.

20. The medical device as claimed in claim 1, wherein said medical device is selected from the group consisting of an IVH catheter, a thermodilution catheter, an angiography catheter, a vasodilation catheter, an artificial blood vessel, a shunt tube, a canula, a dilator, an indwelling needle, a guide wire, a sensor for measuring blood flow or blood gas concentration, a ureteral catheter, a tracheotomy tube, an endotracheal tube, a nasal feeding tube, a nutrition catheter, a feeding gastric tube, an artificial kidney, an artificial heart, an artificial valve and an artificial lung.
